# EUROPEAN PATENT APPLICATION

(11) **EP 1 442 790 A1**
(43) Date of publication of application: **04.08.2004**
(21) Application number: 04250457.1
(22) Date of filing: 28.01.2004
(51) Int. Cl.: B01J 19/00

(54) **Viscosity control during polynucleotide synthesis**

(30) Priority: 31.01.2003 US 355433
(71) Applicant: Agilent Technologies, Inc., Palo Alto, CA 94306 (US)
(72) Inventor: LeProust, Eric M., Campbell, CA 95008 (US); Peck, Bill J., Mountain View, CA 94043 (US); Roltman, Daniel B., Menlo Park, Ca 94025 (US)
(74) Representative: Tollett, Ian

(57) **Abstract**

A method of fabricating an array (12) of biopolymer probes bound to a surface (11a) of a substrate (10). The method includes depositing drops, at least some of which contain probe precursors, onto the substrate surface so that the probe precursors bind to the surface through a linker. This can be repeated multiple times with the probe precursor deposited in a prior cycle becomes the linker for a probe precursor deposited in a subsequent cycle, so as to form the array. The deposited drops may have a viscosity modifier or the viscosities of them may be otherwise controlled as discussed herein. Kits and compositions are further provided.

## Description

This invention relates to methods of fabricating an array bound to a surface of a substrate, particularly polynucleotide arrays such as DNA arrays, which are useful in diagnostic, screening, gene expression analysis, and other applications. It also relates to compositions and kits for such methods.

Polynucleotide arrays (such as DNA or RNA arrays), are known and are used, for example, as diagnostic or screening tools. Such arrays include regions of usually different sequence polynucleotides arranged in a predetermined configuration on a substrate. These regions (sometimes referenced as "features") are positioned at respective locations ("addresses") on the substrate. The arrays, when exposed to a sample, will exhibit an observed binding pattern. This binding pattern can be detected upon interrogating the array. For example all polynucleotide targets (for example, DNA) in the sample can be labeled with a suitable label (such as a fluorescent compound), and the fluorescence pattern on the array accurately observed following exposure to the sample. Assuming that the different sequence polynucleotides were correctly deposited in accordance with the predetermined configuration, then the observed binding pattern will be indicative of the presence and/or concentration of one or more polynucleotide components of the sample.

Biopolymer arrays can be fabricated by depositing previously obtained biopolymers onto a substrate, or by *in situ* synthesis methods. *The in situ* fabrication methods include those described in US 5,449,754 for synthesizing peptide arrays, and in US 6,180,351 and WO 98/41531 and the references cited therein for synthesizing polynucleotide arrays. The *in situ* method for fabricating a polynucleotide array typically follows, at each of the multiple different addresses at which features are to be formed, the same conventional iterative sequence used in forming polynucleotides on a support by means of known chemistry. Typically these methods use a nucleoside reagent of the formula: 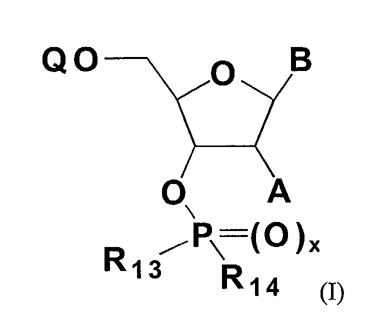 in which:
A represents H, alkyl, or another substituent which does not interfere in the coupling of compounds of formula (I) to form polynucleotides according to the *in situ* fabrication process;
B is a purine or pyrimidine base whose exocyclic amine functional group is optionally protected;
Q is a conventional protective group for the 5'--OH functional group;
x=0 or 1 provided:
   a) when x=1:
      R₁₃ represents H and R₁₄ represents a negatively charged oxygen atom; or
      R₁₃ is an oxygen atom and R₁₄ represents either an oxygen atom or an oxygen atom carrying a protecting group; and
   b) when x=0, R₁₃ is an oxygen atom carrying a protecting group and R₁₄ is either a hydrogen or a di-substituted amine group.

   When x is equal to 1, R₁₃ is an oxygen atom and R₁₄ is an oxygen atom, the method is in this case the so-called phosphodiester method; when R₁₄ is an oxygen atom carrying a protecting group, the method is in this case the so-called phosphotriester method.
   When x is equal to 1, R₁₃ is a hydrogen atom and R₁₄ is a negatively charged oxygen atom, the method is known as the H-phosphonate method.
   When x is equal to 0, R₁₃ is an oxygen atom carrying a protecting group and R₁₄ is either a halogen, the method is known as the phosphite method and; when x = 0, R₁₃ is an oxygen atom carrying a protecting group, and R₁₄ is a leaving group of the disubstituted amine type, the method is known as the phosphoramidite method.

The conventional sequence used to prepare an oligonucleotide using reagents of the type of formula (I), basically follows the following steps: (a) coupling a selected nucleoside through a phosphite linkage to a functionalized support in the first iteration, or a nucleoside bound to the substrate (i.e. the nucleoside-modified substrate) in subsequent iterations; (b) optionally blocking ("capping") unreacted hydroxyl groups on the substrate bound nucleoside; (c) oxidizing the phosphite linkage of step (a) to form a phosphate linkage; and (d) removing the protecting group ("deprotection") from the now substrate bound nucleoside coupled in step (a), to generate a reactive site for the next cycle in which these steps are repeated. The functionalized support (in the first cycle) or deprotected coupled nucleoside (in subsequent cycles) provides a substrate bound moiety with a linking group for forming the phosphite linkage with a next nucleoside to be coupled in step (a). Final deprotection of nucleoside bases can be accomplished using alkaline conditions such as ammonium hydroxide, in a known manner. The nucleoside reagent in (a) generally requires activation by a suitable activator such as tetrazole.

The foregoing methods of preparing polynucleotides are described in detail, for example, in Caruthers, Science 230: 281-285, 1985; Itakura et al., Ann. Rev. Biochem. 53: 323-356; Hunkapillar et al., Nature 310: 105-110, 1984; and in "Synthesis of Oligonucleotide Derivatives in Design and Targeted Reaction of Oligonucleotide Derivatives, CRC Press, Boca Raton, Fla., pages 100 et seq., US 4,458,066, US 4,500,707, US 5,153,319, US 5,869,643, EP 0294196, and elsewhere The phosphoramidite and phosphite triester approaches are most broadly used, but other approaches include the phosphodiester approach, the phosphotriester approach and the H-phosphonate approach.

In the case of array fabrication, different monomers may be deposited at different addresses on the substrate during any one iteration so that the different features of the completed array will have different desired biopolymer sequences. One or more intermediate further steps may be required in each iteration, such as the conventional oxidation and washing steps. One particularly useful way of depositing monomers is by depositing drops each containing a monomer from a pulse jet spaced apart from the substrate surface, onto the substrate surface. Such techniques are described in detail in, for example, US 6,242,266, US 6,232,072, US 6,180,351, and US 6,171,797. Prior art pulse jets are available commercially for use in ink printing which are provided with an indicated viscosity or viscosity range at which the pulse jet will function best. If a liquid with too low a viscosity is used in a pulse jet, transient fluid motion (such as may result from the firing of other pulse jets in a head containing multiple piezo activated pulse jets) will not be sufficiently dampened and other small drops may be ejected from a pulse jet between firing of that pulse jet. In prior art *in situ* techniques for polynucleotide array fabrication drops of five different solutions were deposited, each containing a propylene carbonate solution saturated with one of the four nucleoside phosphoramidites (concentration about 340 mM) or saturated with tetrazole (concentration about 1500 mM) in propylene carbonate. Pure propylene carbonate has a viscosity of about 2.8 cps while each of the foregoing solutions had a viscosity ranging from about 6 to 9 cps (except for tetrazol) depending on the reagent (that is, which phosphoramidite or tetrazole was present in the solution), as illustrated in the following Table:

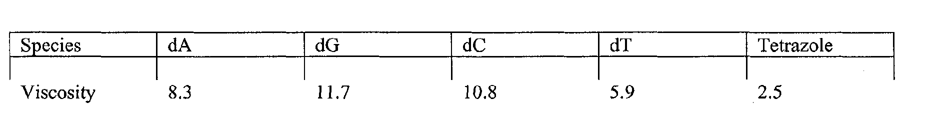

In the above Table the viscosity of the solutions was measured at 25 C using a cone and plate viscometer for shear rates close to 400 s⁻¹. Thus, the presence of the phosphoramidites themselves can raise viscosity. In array fabrication, the probes formed at each feature are usually expensive. Additionally, sample quantities available for testing are usually also very small and it is therefore desirable to simultaneously test the same sample against a large number of different probes on an array. These conditions make it desirable to produce arrays with large numbers of very small, closely spaced features. To facilitate correct interpretation of the data from such arrays, it is important that the features have the characteristics of actually being present at the expected location, and that the different features have the expected size and uniformity.

It is desirable then to provide an array fabrication process which reliably provides good results in terms of feature presence at the expected location, and expected feature size and uniformity.

The present invention recognizes that in array fabrication not only is it important to control drop viscosity to avoid undesired drop ejection (and hence low quality arrays), but also that the control of viscosity by a means of using reagent concentration can lead to other problems. For example, high reagent concentrations can lead to particulate growth inside the pulse jet or at its orifice, leading to unreliable pulse jet operation. Furthermore, different reagents (such as different nucleoside phosphoramidites) may produce solutions with different viscosities at a same concentration which makes viscosity control based on reagent concentration difficult without varying the concentration of the different reagents. The present invention therefore recognizes that it would be desirable in array fabrication to provide a means for controlling fluid viscosity which does not necessarily depend on reagent type or concentration, and which does not seriously affect the yield from each cycle of an *in situ* array fabrication process.

Accordingly, the present invention provides a method of fabricating an array of biopolymer probes bound to a surface of a substrate. One aspect of the method includes depositing drops, at least some of which contain probe precursors, onto the substrate surface so that the probe precursors bind to the surface through a linker. The foregoing depositing is repeated multiple times wherein the probe precursor deposited in a prior cycle becomes the linker for a probe precursor deposited in a subsequent cycle, so as to form the array. The deposited drops include a viscosity modifier, such as a viscosity enhancer in the form of an unblocked-hydroxy free and unblocked-amino free polymer to enhance viscosity. In another aspect of the method of fabricating an array of biopolymers, drops which contain probe precursors are deposited onto the substrate surface so that the probe precursors bind to the surface through a linker. This is repeated multiple times wherein the probe precursor deposited in a prior cycle becomes the linker for a probe precursor deposited in a subsequent cycle, so as to form the array. The viscosity of the deposited drops is above 10 cps and, when only the viscosity enhancer is absent, is below 9 cps (that is, the presence of the viscosity enhancer raises the viscosity by more than 1 cps).

In a further aspect of the method of fabricating an array of biopolymers, the drop depositing and repeating are performed as described above. In this aspect though, different drops containing different probe precursors at a same concentration are deposited at different locations on the substrate surface during one or more cycles. The viscosities of the different drops containing different probe precursors at the same concentration are all within 3 cps of one another.

The present invention further provides a composition including a solvent, a probe precursor dissolved in the solvent, and a viscosity modifier. The viscosity modifier alters the viscosity of the composition by at least 2 cps versus the same composition without only the viscosity modifier.

While the above specifically references biopolymers, it will be understood throughout this application that any desired polymer could be fabricated by methods of the present invention and accordingly, "biopolymer" can generally be replaced by polymer in the description herein (except with regard to the definition of "biopolymer" and the like).

Different various aspects of the present invention can provide any one or more of the following or other useful benefits in biopolymer array fabrication. For example, control of solution viscosity is possible without reliance on reagent concentration. This allows for reagent concentration to be optimized while still obtaining the desired drop viscosity. This also allows for different drops to have the same reagent concentrations (such as probe precursor concentrations) while still having the same viscosity. Viscosity control can be obtained while not substantially adversely affecting the yield during each cycle of the *in situ* process.

A number of preferred embodiments of the invention will now be described with reference to the following drawings in which:
FIGS. 1-5 illustrate properties of different solutions containing a viscosity enhancer and biomonomer, as described further below;
FIG. 6 illustrates a substrate carrying multiple arrays, such as may be fabricated by methods of the present invention;
FIG. 7 is an enlarged view of a portion of FIG. 6 showing multiple spots or features of one array; and
FIG. 8 is an enlarged illustration of a portion of the substrate of FIG. 6;
To facilitate understanding, identical reference numerals have been used, where practical, to designate the same elements which are common to different figures. Drawings are not necessarily to scale. Throughout this application any different members of a generic class may have the same reference number followed by different letters (for example, arrays 12a, 12b, 12c, and 12d may generically be referenced as "arrays 12")

Throughout the present application, unless a contrary intention appears, the following terms refer to the indicated characteristics.

A "biopolymer" is a polymer of one or more types of repeating units. Biopolymers are typically found in biological systems and particularly include polysaccharides (such as carbohydrates), and peptides (which term is used to include polypeptides, and proteins whether or not attached to a polysaccharide) and polynucleotides as well as their analogs such as those compounds composed of or containing amino acid analogs or non-amino acid groups, or nucleotide analogs or non-nucleotide groups. This includes polynucleotides in which the conventional backbone has been replaced with a non-naturally occurring or synthetic backbone, and nucleic acids (or synthetic or naturally occurring analogs) in which one or more of the conventional bases has been replaced with a group (natural or synthetic) capable of participating in Watson-Crick type hydrogen bonding interactions. Polynucleotides include single or multiple stranded configurations, where one or more of the strands may or may not be completely aligned with another. Specifically, a "biopolymer" includes DNA (including cDNA), RNA and oligonucleotides, regardless of the source.

A "biomonomer" references a single unit, which can be linked with the same or other biomonomers to form a biopolymer (for example, a single amino acid or nucleotide with two linking groups one or both of which may have removable protecting groups). A biomonomer fluid or biopolymer fluid reference a liquid containing either a biomonomer or biopolymer, respectively (typically in solution).

A "nucleotide" refers to a sub-unit of a nucleic acid and has a phosphate group, a 5 carbon sugar and a nitrogen containing base, as well as functional analogs (whether synthetic or naturally occurring) of such sub-units which in the polymer form (as a polynucleotide) can hybridize with naturally occurring polynucleotides in a sequence specific manner analogous to that of two naturally occurring polynucleotides.

A "nucleoside" is the same as a nucleotide but without the phosphate group present (for example, there may instead be a phosphite present).

An "oligonucleotide" generally refers to a nucleotide multimer of about 10 to 100 nucleotides in length, while a "polynucleotide" includes a nucleotide multimer having any number of nucleotides.

An "array", unless a contrary intention appears, includes any one, two or three-dimensional arrangement of addressable regions bearing a particular chemical moiety or moieties (for example, biopolymers such as polynucleotide sequences) associated with that region. Each region may extend into a third dimension in the case where the substrate is porous while not having any substantial third dimension measurement (thickness) in the case where the substrate is non-porous. An array is "addressable" in that it has multiple regions of different moieties (for example, different polynucleotide sequences) such that a region (a "feature" or "spot" of the array) at a particular predetermined location (an "address") on the array will detect a particular target or class of targets (although a feature may incidentally detect non-targets of that feature). An array feature is generally homogenous and the features typically, but need not be, separated by intervening spaces. In the case of an array, the "target" will be referenced as a moiety in a mobile phase (typically fluid), to be detected by probes ("target probes") which are bound to the substrate at the various regions. However, either of the "target" or "target probes" may be the one which is to be evaluated by the other (thus, either one could be an unknown mixture of polynucleotides to be evaluated by binding with the other).

An "array layout" or "array characteristics", refers to one or more physical, chemical or biological characteristics of the array, such as feature positioning, one or more feature dimensions, or some indication of an identity or function (for example, chemical or biological) of a moiety at a given location, or how the array should be handled (for example, conditions under which the array is exposed to a sample, or array reading specifications or controls following sample exposure).

"Hybridizing" and "binding", with respect to polynucleotides, are used interchangeably.

A "plastic" is any synthetic organic polymer of high molecular weight (for example at least 1,000 grams/mole, or even at least 10,000 or 100,000 grams/mole.

"Flexible" with reference to a substrate or substrate web, references that the substrate can be bent 180 degrees around a roller of less than 1.25 cm in radius. The substrate can be so bent and straightened repeatedly in either direction at least 100 times without failure (for example, cracking) or plastic deformation. This bending must be within the elastic limits of the material. The foregoing test for flexibility is performed at a temperature of 20 °C.

A "web" references a long continuous piece of substrate material having a length greater than a width. For example, the web length to width ratio may be at least 5/1, 10/1, 50/1, 100/1, 200/1, or 500/1, or even at least 1000/1.

When one item is indicated as being "remote" from another, this is referenced that the two items are at least in different buildings, and may be at least one mile, ten miles, or at least one hundred miles apart. "Communicating" information references transmitting the data representing that information as electrical signals over a suitable communication channel (for example, a private or public network). "Forwarding" an item refers to any means of getting that item from one location to the next, whether by physically transporting that item or using other known methods (where that is possible) and includes, at least in the case of data, physically transporting a medium carrying the data or communicating the data over a communication channel (including electrical, optical, or wireless).

An array "assembly" may be the array plus only a substrate on which the array is deposited, although the assembly may be in the form of a package which includes other features (such as a housing with a chamber). A "chamber" references an enclosed volume (although a chamber may be accessible through one or more ports).

It will also be appreciated that throughout the present application, that words such as "front", "back", "top", "upper", and "lower" are used in a relative sense only.

A "pulse jet" is any device which can dispense drops in the formation of an array. Pulse jets operate by delivering a pulse of pressure (such as by a piezoelectric or thermoelectric element) to liquid adjacent an outlet or orifice such that a drop will be dispensed therefrom.

A "group" in relation to a chemical formula, includes both substituted and unsubstituted forms of the group where any substituents do not interfere with the desired reactions.

A "phospho" group includes a phosphodiester, phosphotriester, and H-phosphonate groups as defined in connection with formula (I) above, while a "phosphite" includes a phosphoramidite.

A "phosphoramidite" is a compound of formula (I) when x=0 and R₁₄ is a leaving group of the disubstituted amine type. A "nucleoside phosphoramidite" is a nucleoside having a phosphoramidite group such as at the 3' or 5' position on the furyl ring in formula (I).

"Lower alkyl group" or other "lower" group references either such group with from 1 to 6 C atoms (such as 2, 3, 4, or 5 C atoms).

A "blocked" hydroxy group references a hydroxy group ( -OH ) in which the free H has been replaced by a protecting group which renders the hydroxy unreactive under the conditions of an *in situ* biopolymer fabrication process in which it is used.

A "blocked polyhydric polymer" is any polymer in which the polymer molecules each has multiple blocked hydroxy groups. An example of a blocked polyhydric polymer is a blocked polyalkylene glycol which can be thought of as a polymer of alkylene glycol units (such as a lower alkylene glycol, for example ethylene glycol).

"Unblocked-hydroxy free" and unblocked-amino free" polymers refer to polymers which do not have any unblocked hydroxy or amino groups (that is, do not have an -OH or an -NR¹H group, where R¹ may be H or another substituent).

By "same concentration" of different solutions is referenced that the concentrations, on a molar basis, are within 20% of one another (although they may be within 15, 10, 5 or 2% of one another).

A "molecular weight" of a polymer which may be a mixture of polymers, is taken as the average molecular weight of the mixture. Molar concentrations of such mixtures are based on the average molecular weight.

"Fluid" is used herein to reference a liquid.

"May" refers to optionally.

All viscosities herein are in centipoise ("cp") at 25°C unless otherwise noted and are based on a rotational rheometer at a shear rate of 400 sec⁻¹.

Any recited method can be carried out in the order of events recited or in any other order which is logically possible. Reference to a singular item, includes the possibility that there are plural of the same item present. All patents and other references cited in this application, are incorporated into this application by reference except insofar as anything in those patents or references, including definitions, conflicts with anything in the present application (in which case the present application is to prevail).

In any method of the present invention, the viscosity enhancing polymer may be an unblocked-hydroxy free and unblocked-amino free polymer, such as a blocked polyhydric polymer. For example, the polymer may be a blocked polyalkylene glycol such as one having the formula II below:

B¹-O-(Alk-O)ₙ-Alk-O-B² (II)

where B¹ and B² are blocking groups which may be the same or different, n is an integer (such as 2 to 10000, for example or 100 to 6000 or 200 to 3000), and Alk is an alkylene group (such as a lower alkylene group, for example ethylene (-CH₂-CH₂-)). Note that in formula (II) both terminal hydroxy groups are blocked by blocking groups B¹ or B² although some small number of the molecules may not have both hydroxy groups blocked provided any resulting small reduction in coupled product yield can be tolerated.

Suitable blocking groups for any hydroxy or amine may be any group that does not allow the blocked O or N to react with the probe precursor (or precursors where more than one is present) in the same liquid under the conditions used (for example, presence of tetrazole activator) to link one probe precursor to another on a substrate surface, or in any other manner does not adversely affect the yield of coupled product resulting from using that liquid in a cycle of the *in situ* probe synthesis. By "not adversely affecting" yield is referenced that any reduction in yield is less than 5%, 4%, 3%, 2%, 1% or 0.5% or even less than 0.2%. The foregoing percentages are based on a theoretical maximum of 100% such that if the yield without a compound of formula (II) was 98%, then the yield would not be less than 93% in the extreme case where yield is reduced by 5%. Suitable blocking groups for use in typical *in situ* probe synthesis methods, such as phosphoramidite chemistry, include hydrocarbyl radicals, such as alkyl (for example lower alkyl groups) or aryl (for example, benzyl); or ester groups (for example, -C(O)OR6 or -OC(O)R6 where R6 is an alkyl or aryl group such as a lower alkyl group); or ether groups (for example, -C-O-R6 where R6 is an alkyl or aryl group such as a lower alkyl group). Other blocking groups which may be used include any of those protecting groups used to protect the 3' or 5' hydroxy of a nucleoside phosphoramidite during linking to the hydroxy of a previously deposited nucleoside phosphoramidite or on the surface. Such blocking groups may be protecting groups such as described in "Protective groups in organic synthesis" by Theodora W. Greene and Peter G. M. Wuts , Wiley-interscience ISBN 0-471-62301-6 p.68-117, and may be made by methods described therein or otherwise.

The deposited drops in any method of the present invention may include probe precursors or alternatively another reagent such as an activator (for example, tetrazole) to facilitate the linking of one probe precursor to a previously deposited probe precursor. The viscosity of the probe precursor containing drops when only the viscosity enhancer is absent (that is, the viscosity as measured on the drops of the same composition but without only the viscosity enhancer) may be below 9 cps or even below 5 cps.

The amount of polymer present may be varied but may be sufficient to raise the viscosity of the drops (versus their viscosity without only the viscosity enhancer) by at least 2, 3, or 4 cps or even 5, 7, or 8 cps. The viscosity of the drops (which means with the viscosity enhancer present) may be at least 7 cps, or even above 9, 10, 11, or 13 cps. Regardless of the absolute viscosities of the drops, the viscosities of the different drops containing different probe precursors at the same concentration may all be within 4, 3, 2, 1 or 0.5 cps of one another. Such different drops may have viscosities which are not all within 4 or 3 cps of one another absent the viscosity modifier (that is, if only the viscosity modifier was removed from the different drops). Also, while generally one would want to have a high as viscosity as possible while still maintaining a high reliability and uniformity of deposited drops (as discussed below), typically the viscosity of the drops (with viscosity modifier present) will not exceed 30, cps.

Other viscosity enhancers might be used instead of those described above. Furthermore, while viscosity enhancers have been primarily discussed above, the present invention allows for the use of viscosity modifiers (including viscosity reducers) in general. Again, if these groups have any hydroxy or amine present these should be blocked. Suitable blocking groups should meet the conditions already discussed above. Any viscosity modifier should be compatible with the chemistry in which it is to be used so that it does not adversely affect coupling yield (as defined above). The viscosity modifier will also be soluble in the liquid containing the probe precursor.

The concentrations of the probe precursors in the drops may be varied as desired and may be less than 4 440, 420, 400, 380, 360, or 340 mM, or even less than 300, 250, 200, or 150 mM. The concentration of the activator in the drops may be varied as desired and maybe less than 1500, 1300, 1100, 900, 800, 600 mM, or even less than 400, 300, 200, or 100 mM.

Various solvents may be used in the drops, such as any of those described in US 6,028,189, US 6,384,210, and US 6,419,883. Particular solvents may include an alkylene carbonate solvent, such as propylene carbonate. The biopolymer probes may particularly be polynucleotides and the probe precursors are nucleoside phosphoramidites.

Probe precursors may be any biomonomer, such as a nucleoside monomer (for example, a nucleoside phosphoramidite) or amino acid monomer, which has first and second linking groups such that a polynucleotide or peptide probe can be formed by a method which includes sequential deposit of different probe precursors onto a surface in different cycles. Such methods and suitable linking groups are described above and in the cited references, in relation to the *in situ* fabrication methods.

Liquid compositions of probe precursor and viscosity modifier as described herein, are readily prepared by first selecting a solvent with a desired concentration of probe precursor. Viscosity of this composition may be measured and a viscosity modifier added as needed to alter the measured viscosity to a figure indicated as suitable by a pulse jet manufacturer. The solution of the desired viscosity can then be tested in the pulse jet head to be used, both for reliability and uniformity of drop deposition. Reliability can be determined by repeatedly depositing drops from the same pulse jet over a long period of time (for example, 24 hours) and checking the number of drops which were in fact deposited. Uniformity can be determined by examining the drops deposited over time for size uniformity and for the presence of any deposited satellite drops, such as by capturing images of deposited drops with a linescan or other camera of suitable resolution and either manually examining the images or using image processing techniques to compare deposited drop sizes. If the results are unsatisfactory, viscosity can be adjusted and the testing in the pulse jet head repeated. Such actual testing in the pulse jet head to be used reduces problems where the liquid being used is non-Newtonian (that is, the viscosity issubstantially dependent on shear rate) and the viscosity measuring device is incapable of measuring viscosities at the shear rates in a typical piezo activated pulse jet. For example, a typical viscosity measuring device may only be able to reach shear rates of between 1- 1800 sec⁻¹ whereas shear rates in a piezo activated pulse jet may be about 1 million sec⁻¹. Alternatively, a suitable initial viscosity can just be estimated (for example, start with 6 cps) and the testing in the pulse jet, viscosity adjustment, and repeated testing in the pulse jet, performed as before. An upper and lower limit of suitable viscosity for a particular pulse jet can be determined in this manner. Compatibility of the viscosity modifier with the chemistry may be tested by comparing the stepwise coupling yield of the nucleotide precursor in solution of the same liquid composition both with and without the presence of the viscosity modifier.

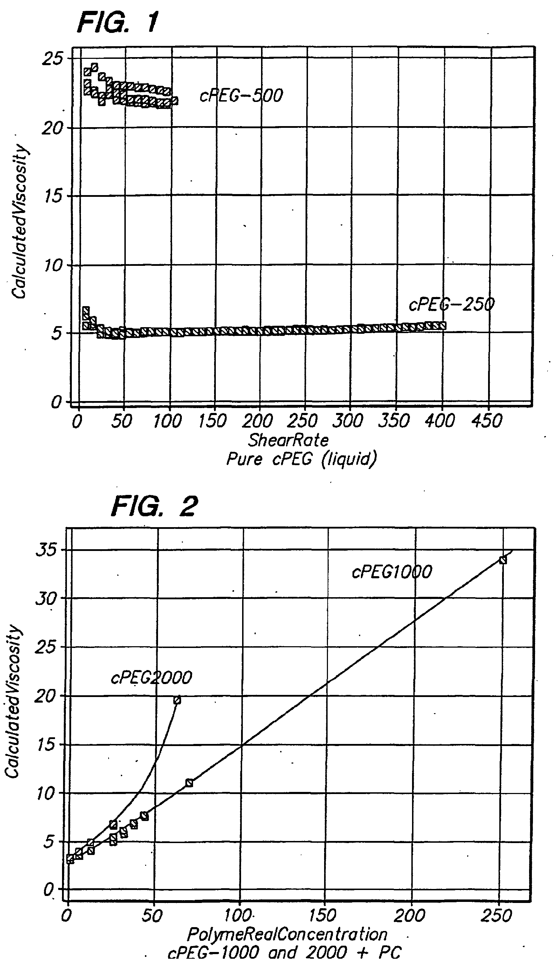

Viscosity enhancers composed of blocked poleythylene glycol, of various average molecular weights, were prepared in which the terminal hydroxy groups were blocked by methyl. This product was commercially obtained from Sigma Aldrich, MO, U.S.A. It is believed that this product contains some small proportion of molecules with unblocked terminal hydroxy, however the affect on yield was considered acceptable. Each solution to be used should be checked for any effect in reducing coupling yield as it was found that some capped PEG samples of average molecular weight of 500 and 250 did not work. Solutions were prepared of the various blocked polyethylene glycols (referenced as "cPEG") with nucleoside phosphoramidites. The nucleoside phosphoramidites are of formula (I) above in which A is H; x = 0; R₁₃ is a protected oxygen in the form of -OCH₂CH₂CN; R₁₄ is -N(isopropyl)₂; the protecting group Q is trityl; and B is any of the usual four protected purine or pyrimidine bases of nucleosides Viscosity characteristics of the various solutions were measured and the results illustrated in FIGS. 1-5. In FIGS. 1-5: "cPEG" is the blocked polyethylene glycol and the number following "cPEG" is the average molecular weight (for example, "cPEG-500" is blocked polyethylene glycol with an average molecular weight of 500 g/mole); "Calculated Viscosity" is calculated based on torque in a rotational rheometer (model DV-III from Brookfield Instruments) at a maximum shear rate available from the instrument (the instrument is capable of shear rates of from close to 0 to 2000sec⁻¹ depending on the solution). However, since these solutions were found to be essentially Newtonian the calculated viscosity can be taken as representative of the viscosity at 400 sec⁻¹); "PC" is the propylene carbonate solvent; "PA" is one of the nucleoside phosphoramidites (with dA, dC, dG, dT representing the usual four nucleoside phosphoramidites where B in formula (I) is adenine for dA, cytosine for dC, guanine for dG, and thymine for dT); "Tet" is tretrazole; the concentrations are shown as weight percentages of cPEG per unit volume of solution, while the concentrations of or PA or Tet are in mM. Many of the measurements were repeated a number of times as indicated by adjacent points on the plots of FIGS. 1-5

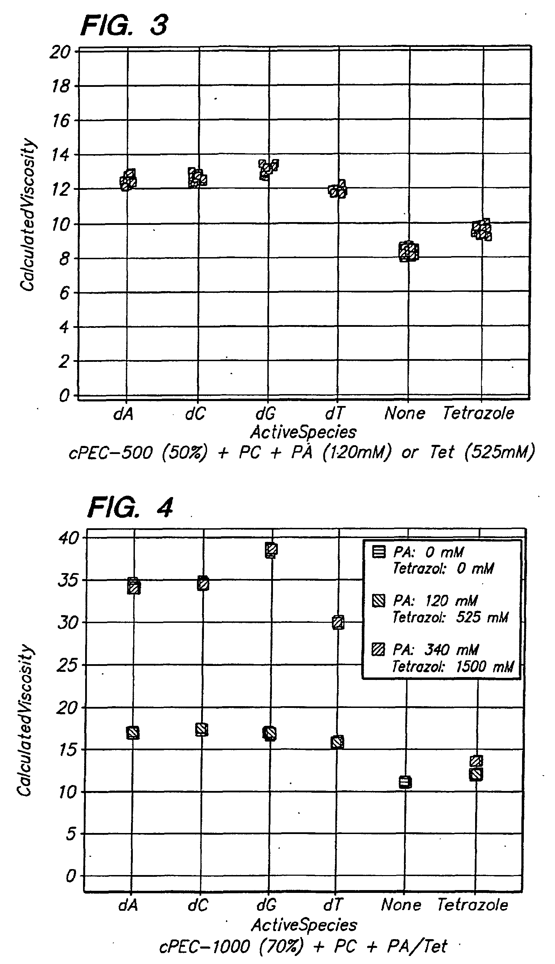

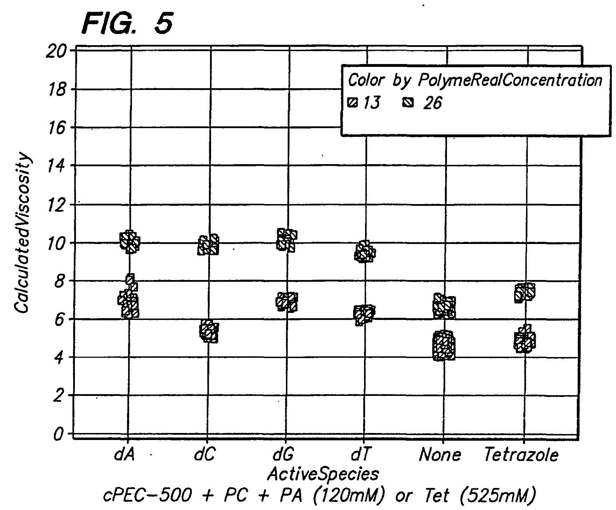

Referring to FIG. 1 it will be seen that for cPEG viscosity of cPEG-250 or cPEG-500 remains relatively constant at different shear rates. In each case the maximum shear rate available from the rheometer was reached at the end of the plots. These solutions then were essentially Newtonian in their viscosity characteristics as was the case for the other solutions in FIGS. 1 - 5. FIG. 2 illustrates variation of viscosity of different average molecular weight cPEG with change in concentration. Note that in both cases viscosity increases with increasing concentration with cPEG-1-1000 providing more linear performance. FIG. 3 shows that with a 50% cPEG-500 concentration and a 120 mM PA or 525 mM Tet concentration, viscosity of the different PA containing solutions of the same concentration will fall within a range suitable for many pulse jets (greater than about 8 or 10 cps), but still varies somewhat (all PA solutions falling within a range of about 1 cps) but not as much as would be the case absent the cPEG. Also it can be seen from FIG. 3 that while each of the PAs present raises the viscosity of the overall solution by about 4 to 5 cps, tetrazole has a much lower effect on viscosity. FIG. 4 illustrates that 70% cPEG-1000 can also effectively raise the viscosity of 120 mM PA and 525 mM tetrazole solutions to a value (greater than 10 cps and less than 20 cps) that will be suitable for many pulse jets. Note that the same amount of cPEG-1000 raises a saturated PA solution (approximately 340 mM) to values that are probably too high for many pulse jets (greater than 30 cps). FIG. 4 also demonstrates how the cPEG viscosity enhancer allows lower concentrations of the polynucleotide precursors (the PA) to be used while still maintaining a high viscosity. FIG. 5 on the other hand illustrates that an even lower concentration (26%) of cPEG-2000 can produce viscosities (greater than 8 or 9 cps) that may be acceptable for some pulse jets while a concentration of 13% provides viscosities which may be too low for some pulse jets. For comparison, the viscosities of the various phosphoramide and tetrazole solutions in propylene carbonate solvent, but without cPEG or other added viscosity enhancer present, are as follows (solutions are 120 mM for each of the phosphoramidites and 525 mM for tetrazol):

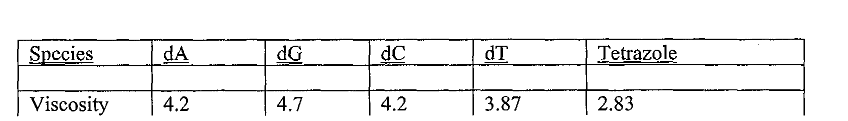

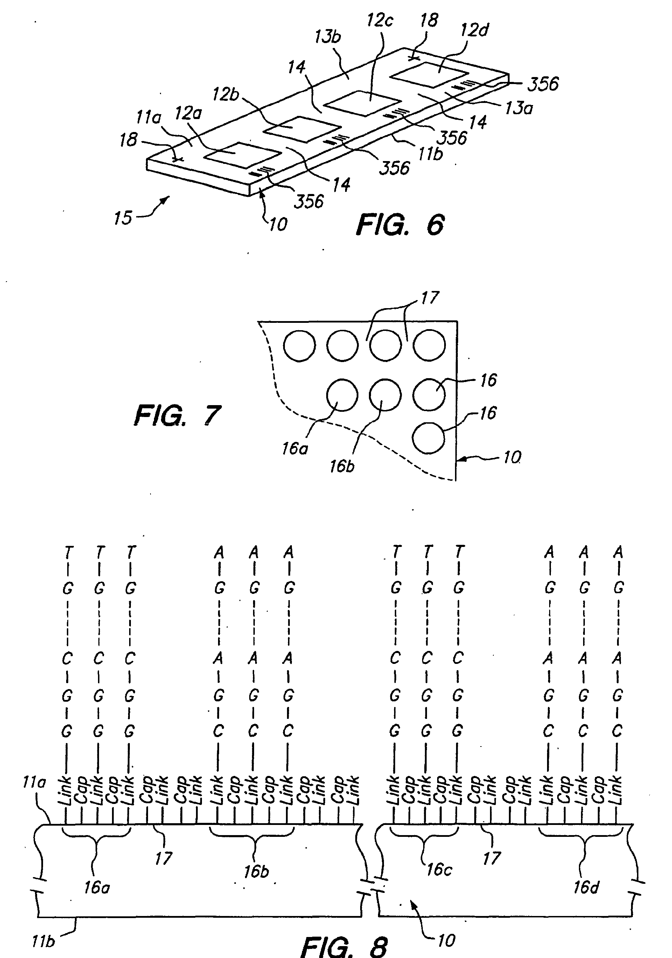

Referring now to FIGS. 6-8, an array assembly 15 (which may be referenced also as an "array unit") fabricated by a method of the present invention may include a porous or non-porous substrate which may be smooth or substantially planar, or have irregularities, such as depressions or elevations (although irregular substrate surfaces may make reading of the exposed array more difficult). Substrate 10 may also be in the form of an a rigid substrate 10 (for example, a transparent non-porous material such as glass or silica) of limited length, carrying one or more arrays 12 disposed along a front surface 11a of substrate 10 and separated by inter-array areas 14. Alternatively, substrate 10 can be flexible (such as a flexible web). The substrate may be of one material or of multi-layer construction. A back side 11b of substrate 10 does not carry any arrays 12. The arrays on substrate 10 can be designed for testing against any type of sample, whether: a trial sample; reference sample; a combination of the foregoing; or a known mixture of polynucleotides, proteins, polysaccharides and the like (in which case the arrays may be composed of features carrying unknown sequences to be evaluated). While four arrays 12 are shown in FIG. 6, it will be understood that substrate 10 and the embodiments to be used with it, may use any number of desired arrays 12 such as at least one, two, five, ten, twenty, fifty, or one hundred (or even at least five hundred, one thousand, or at least three thousand). When more than one array 12 is present they may be arranged end to end along the lengthwise direction of substrate 10. Depending upon intended use, any or all of arrays 12 may be the same or different from one another and each will contain multiple spots or features 16 of biopolymers in the form of polynucleotides.

A typical array 12 may contain from more than ten, more than one hundred, more than one thousand or ten thousand features, or even more than from one hundred thousand features. For example, features may have widths (that is, diameter, for a round spot) in the range from a 10 µm to 1.0 cm. In other embodiments each feature may have a width in the range of 1.0 µm to 1.0 mm, usually 5.0 µm to 500 µm, and more usually 10 µm to 200 µm. Non-round features may have area ranges equivalent to that of circular features with the foregoing width (diameter) ranges. At least some, or all, of the features are of different compositions (for example, when any repeats of each feature of the same composition are excluded, the remaining features may account for at least 5%, 10%, or 20% of the total number of features).

In any aspect of the present invention, the features 16 may be spaced apart by a distance greater than 0 and less than 70%, 60% 50%, 25%, or 10% of a maximum dimension of the feature. Further, the features may have a maximum dimension of between 20 (or 50) to 100 (or 80) microns and are spaced apart by less than 130 microns (or by less than 100 or 50 microns). Various feature densities on the substrate surface are possible. For example, features having a maximum dimension greater than any of the foregoing figures may be present on the surface of at least 30 features/mm², 40 features/mm², or 60 features/mm². While round features 16 are shown, various other feature shapes are possible (such as elliptical). The features 16 may also be arranged in other configurations (for example, circular) rather than the rectilinear grid illustrated. Similarly, arrays 12 on a same substrate 10 need not be laid out in a linear configuration.

Each array 12 may cover an area of less than 100 cm², or even less than 50 cm², 10 cm² or 1 cm². In many embodiments, particularly when substrate 10 is rigid, it may be shaped generally as a rectangular solid (although other shapes are possible), having a length of more than 4 mm and less than 1 m, usually more than 4 mm and less than 600 mm, more usually less than 400 mm; a width of more than 4 mm and less than 1 m, usually less than 500 mm and more usually less than 400 mm; and a thickness of more than 0.01 mm and less than 5.0 mm, usually more than 0.1 mm and less than 2 mm and more usually more than 0.2 and less than 1 mm. When substrate 10 is flexible, it may be of various lengths including at least 1 m, at least 2 m, or at least 5 m (or even at least 10 m). With arrays that are read by detecting fluorescence, the substrate 10 may be of a material that emits low fluorescence upon illumination with the excitation light. Additionally in this situation, the substrate may be relatively transparent to reduce the absorption of the incident illuminating laser light and subsequent heating if the focused laser beam travels too slowly over a region. For example, substrate 10 may transmit at least 20%, or 50% (or even at least 70%, 90%, or 95%), of the illuminating light incident on the front as may be measured across the entire integrated spectrum of such illuminating light or alternatively at 532 nm or 633 nm.

In the case where arrays 12 are formed by the *conventional in situ* or deposition of previously obtained moieties, as described above, by depositing for each feature a droplet of reagent in each cycle such as by using a pulse jet such as an inkjet type head, interfeature areas 17 will typically be present which do not carry any polynucleotide. It will be appreciated though, that the interfeature areas 17 could be of various sizes and configurations. Each feature 16 carries a predetermined polynucleotide (which includes the possibility of mixtures of polynucleotides). As per usual, A, C, G, T represent the usual four nucleotides. "Link" (see FIG. 8 in particular) represents a linking agent (molecule) covalently bound to the front surface and a first nucleotide, as provided by a method of the present invention and as further described below. The Link serves to functionalize the surface for binding by the first nucleotide during the *in situ* process. "Cap" represents a capping agent. The Link may be any of the "second silanes" referenced in U.S. Patent 6,444,268 while the Cap may be any of the "first silanes" in that patent. However, different linking layer compositions than those silanes could be used. As already mentioned, the foregoing patents are incorporated herein by reference, including for example the details of the linking layer compositions used therein.

Substrate 10 also one or more identifiers in the form of bar codes 356. Identifiers such as other optical or magnetic identifiers could be used instead of bar codes 356 which will carry the information discussed below. Each identifier may be associated with its corresponding array by being positioned adjacent that array 12. However, this need not be the case and identifiers such as bar code 356 can be positioned elsewhere on substrate 10 if some other means of associating each bar code 356 with its corresponding array is provided (for example, by relative physical locations). Further, a single identifier might be provided which is associated with more than one array 12 on a same substrate 10 and such one or more identifiers may be positioned on a leading or trailing end of substrate 10. The substrate may further have one or more fiducial marks 18 for alignment purposes during array fabrication.

FIGS. 7 and 8 illustrate ideal features 16 of an array 12 where the actual features formed are the same as the target (or "aim") features, with each feature 16 being uniform in shape, size and composition, and the features being regularly spaced. Such an array when fabricated by drop deposition methods, would require all reagent droplets for each feature to be uniform in shape and accurately deposited at the target feature location. In practice, such an ideal result may be difficult to obtain due to fixed and random errors during fabrication.

Arrays 12 may be fabricated on the functionalized surface 11a by depositing onto the continuous functionalized area on the substrate surface, drops containing the probe precursors and viscosity modifier, as described above, at the multiple feature locations of the array to be fabricated, so that the probes or probe precursors bind to the linking agent at the feature locations. This step is repeated in subsequent "cycles" at one or more features. Such methods and their chemistry are described in detail in the references cited in the "Background" section above.

A suitable apparatus and *in situ* method for fabricating arrays 12 is described in US 6,180,351, US 6,242,266, US 6,306,599, and US 6,420,180, particularly where each pulse jet of each deposition head uses a piezoelectric element rather than a thermal element for activation to eject a drop. As mentioned above, the foregoing references are incorporated herein by reference particularly as relates to the *in situ* fabrication apparatus and methods disclosed therein. As described in the foregoing a typical same drop deposition head may have multiple pulse jets on it. By a "same head" in the foregoing context is meant one in which the different pulse jets have at least one common unitary component, such as a unitary orifice plate carrying the orifices of different pulse jets or a unitary member which defines walls of the dispensing chambers of different pulse jets. A same head unit may have more than one head on it which heads all move in unison. The head unit will typically simultaneously contain all the different reagent solutions as described above (each solution of different biopolymer precursor or activator with viscosity modifier) required for one or more cycles at all of the features. Other pulse jet heads can of course be used. the amount of fluid that is expelled in a single activation event of a pulse jet, can be controlled by changing one or more of a number of parameters, including the orifice diameter, the orifice length (thickness of the orifice member at the orifice), the size of the deposition chamber, and the size of the heating element, among others. The volume of a drop of fluid that is expelled during a single activation event of a pulse jet is generally in the range about 0.1 to 1000 pL, usually about 0.5 to 500 pL and more usually about 1.0 to 250 pL. A typical velocity at which the fluid is expelled from the chamber is more than about 1 m/s, usually more than about 10 m/s, and may be as great as about 20 m/s or greater. A series of drops of the same composition can be dispensed from a same pulse jet to a same feature location during a same cycle, rather than one larger drop of volume equivalent to the total volume of the series.

In order to execute a method of the present invention, a user may be provided with a kit. This kit may include a set of different compositions as used in a method of the present invention where each of the different compositions comprises a different probe precursor (for example, a different one of four nucleoside phosphoramidites). The kit may also contain a solution of further reagents, such as an activator, plus viscosity enhancer, as described herein. Additionally, the kit may further contain instructions to use the compositions in a piezoelectric pulse jet. These instructions may be for example be in human readable written form. All components of the kit may be contained in a same container and although the instructions might alternatively be provided on or attached to the outside of the container. These compositions may then be used in the foregoing described apparatus and methods to fabricate arrays 12.

Following receipt by a user of an array made according to the present invention, the array will typically be exposed to a sample (for example, a fluorescently labeled polynucleotide or protein containing sample) and the array then read. All arrays 12 on substrate 10 can be read at the same time or not by using any suitable reading apparatus. Where fluorescent light is to be detected due to incorporation of fluorescent labels into the target in a known manner, well known array readers can be used. For example, such a reader may scan one or more illuminating laser beams across each array in raster fashion and any resulting fluorescent signals detected, such as described in US 6,406,849. One such scanner that may be used for this purpose is the AGILENT MICROARRAY SCANNER manufactured by Agilent Technologies, Palo Alto, CA. However, arrays may be read by any other method or apparatus than the foregoing, with other reading methods including other optical techniques (for example, detecting chemiluminescent or electroluminescent labels) or electrical techniques (where each array feature is provided with an electrode to detect hybridization at that feature in a manner disclosed in US 6,251,685, US 6,221,583 and elsewhere).

Results from the array reading can be further processed results, such as obtained by rejecting a reading for a feature which is below a predetermined threshold and/or forming conclusions based on the pattern read from the array (such as whether or not a particular target sequence may have been present in the sample or an organism from which the sample was obtained exhibits a particular condition or disease). The results of the reading (processed or not) can be forwarded (such as by communication) to be received at a remote location for further evaluation and/or processing, or use, using communication channel 180 or reader/writer 186 and medium 190. This data may be transmitted by others as required to reach the remote location, or re-transmitted to elsewhere as desired.

## Claims

1. A method of fabricating an array 12 of biopolymer probes bound to a surface 11 a of a substrate 10, comprising:
(a) depositing drops, at least some of which contain probe precursors, onto the substrate surface so that the probe precursors bind to the surface through a linker; and
(b) repeating (a) multiple times wherein the probe precursor deposited in a prior cycle becomes the linker for a probe precursor deposited in a subsequent cycle, so as to form the array;
wherein the deposited drops comprise an unblocked-hydroxy free and unblocked-amino free polymer to enhance viscosity.

2. A method according to claim 1 wherein the biopolymer probes comprise polynucleotide or peptide probes.

3. A method according to claim 1 or 2 wherein the polymer is a blocked polyhydric polymer.

4. A method according to any preceding claim wherein the drops comprise probe precursors and the viscosity of the probe precursor containing drops when only the viscosity enhancer is absent is below 9 cps.

5. A method according to claim 4 wherein the drops comprise probe precursors and the viscosity of the probe precursor containing drops when only the viscosity enhancer is absent is below 5 cps.

6. A method according to claim 5 wherein sufficient polymer is present to raise the viscosity of the drops comprising probe precursors by at least 2 cps.

7. A method according to claim 6 wherein sufficient polymer is present to raise the viscosity of the drops comprising probe precursors by at least 4 cps.

8. A method according to claim 5 wherein the viscosity of the drops is above 10 cps.

9. A method according to any preceding claim wherein the probe precursors are at a concentration of less than 400 mM.

10. A method according to any preceding claim wherein:
the biopolymer probes comprise polynecleotides and the probe precursors comprise nucleoside phosphoramidites;
different drops containing different probe precursors at a same concentration are deposited at different locations on the substrate surface during one or more cycles; and
the viscosities of the different drops containing different probe precursors at the same concentration are all within 2 cps of one another.

11. A method according to any preceding claim wherein the drops are deposited from different pulse jets in a same pulse jet head.

12. A method according to claim 15 wherein the pulse jets are activated by piezoelectric elements.

13. A method according to any preceding claim additionally comprising exposing the fabricated array to a sample.

14. A method according to claim 18 additionally comprising, following exposure of the array to a sample, reading the array.

15. A method comprising forwarding a result of a reading obtained by a method as claimed in any preceding claim, to a remote location.

16. A method comprising receiving a result of reading obtained by a method as claimed in any preceding claim from a remote location.

17. A method of fabricating an array of biopolymer probes bound to a surface of a substrate, comprising:
(a) depositing drops which contain probe precursors onto the substrate surface so that the probe precursors bind to the surface through a linker; and
(b) repeating (a) multiple times wherein the probe precursor deposited in a prior cycle becomes the linker for a probe precursor deposited in a subsequent cycle, so as to form the array;
wherein viscosity of the drops is above 10 cps and, when only the viscosity enhancer is absent, is below 9 cps.

18. A method according to claim 21 wherein the deposited drops comprise a probe precursor and a viscosity enhancer so as to have a viscosity above 11 cps.

19. A method according to claim 17 or 18 wherein the viscosity enhancer is an unblocked-hydroxy free and unblocked-amino free polymer.

20. A method according to any of claims 1 to 14 or 17 to 19 wherein the drops comprise an alkylene carbonate solvent.

21. A method according to any of claims 1 to 14 or 17 to 20 wherein the probe precursors are nucleoside phosphoramidites.

22. A method of fabricating an array of biopolymer probes bound to a surface of a substrate, comprising:
(a) depositing drops which contain probe precursors onto the substrate surface so that the probe precursors bind to the surface through a linker; and
(b) repeating (a) multiple times wherein the probe precursor deposited in a prior cycle becomes the linker for a probe precursor deposited in a subsequent cycle, so as to form the array;
wherein:
different drops containing different probe precursors at a same concentration are deposited at different locations on the substrate surface during one or more cycles; and
the viscosities of the different drops containing different probe precursors at the same concentration are all within 3 cps of one another.

23. A method according to claim 26 wherein the biopolymer probes comprise polynucleotide or peptide probes.

24. A method according to any of claims 1 to 14 or 17 to 23 wherein the biopolymer probes comprise polynucleotide probes and the probe precursors comprise nucleoside phosphoramidites.

25. A method according to claim 22 or 23 wherein:
the biopolymer probes comprise polynucleotide probes and the probe precursors comprise nucleoside phosphoramidites; and
the viscosities of the different drops containing different probe precursors at the same concentration are all within 2 cps of one another.

26. A method according to claim 29 wherein the different drops containing different probe precursors at the same concentration all additionally comprise a viscosity modifier such that the viscosities of all those drops are not within 3 cps of one another absent the viscosity modifier.

27. A method according to claim 30 wherein the viscosity modifier in at least some of the drops is a viscosity enhancer.

28. A method according to claim 31 wherein the viscosity enhancer is a blocked polyhydric polymer.

29. A method according to claim 32 wherein the polymer is a blocked polyalkylene glycol.

30. A composition comprising:
a solvent;
a probe precursor dissolved in the solvent; and
a viscosity modifier which alters the viscosity of the composition by at least 2 cps versus the same composition without only the viscosity modifier.

31. A composition according to claim 34 wherein the viscosity modifier is an unblocked-hydroxy free and unblocked-amino free polymer to enhance viscosity.

32. A composition according to claim 34 wherein the probe precursor is a nucleoside monomer or amino acid monomer, the monomer having first and second linking groups such that a polynucleotide or peptide probe can be formed by a method comprising sequential deposit of different probe precursors onto a surface in different cycles.

33. A kit comprising a set of different compositions each according to claim 17, wherein each of the different compositions comprises a different probe precursor.

34. A kit according to claim 33 wherein the different probe precursors are different nucleoside phosphoramidites.

35. A method according to any of claims 1 to 14 wherein the polymer to enhance viscosity does not reduce coupling yield in a cycle by more than 5%.
